(19) Europäisches Patentamt
European Patent Office
Office européen des brevets

(11) Numéro de publication : **0 012 674**
**B1**

(12) # FASCICULE DE BREVET EUROPÉEN

(45) Date de publication du fascicule du brevet : **13.07.83**

(51) Int. Cl.$^3$ : **C 07 D519/04**

(21) Numéro de dépôt : **79400976.1**

(22) Date de dépôt : **06.12.79**

(54) **Procédé de synthèse de la vinblastine.**

(30) Priorité : **08.12.78 FR 7834599**

(43) Date de publication de la demande : **25.06.80 Bulletin 80/13**

(45) Mention de la délivrance du brevet : **13.07.83 Bulletin 83/28**

(84) Etats contractants désignés : **BE CH DE GB IT NL**

(56) Documents cités :
**FR A 2 296 418**

**TETRAHEDRON LETTERS, no. 14 (1976) Oxford, GB N. LANGLOIS et al. : « Hémisynthèse de la leurosidine, alcaloïde antitumoral isolé de catharanthus roseus G. Don (Apocynacées) » Pages 1099-1102**

**Houben-Weyl, Band IV/1b pages 119-122 ; Reagents for Organic Synthesis, LF Fieser, 1967 pages 1150, 1151**

(73) Titulaire : **ANVAR Agence Nationale de Valorisation de la Recherche**
**13, rue Madeleine Michelis**
**F-92522 Neuilly-sur-Seine (FR)**

(72) Inventeur : **Potier, Pierre**
**5, rue de la Fontaine**
**F-78390 Bois d'Arcy (FR)**
Inventeur : **Mangeney, Pierre**
**10, rue de l'Ingénieur Keller**
**F-75015 Paris (FR)**
Inventeur : **Langlois, Nicole**
**32, rue de Gometz**
**F-91440 Bures S. Yvette (FR)**
Inventeur : **Langlois, Yves**
**32, rue de Gometz**
**F-91440 Bures S. Yvette (FR)**

(74) Mandataire : **Corre, Jacques Denis Paul et al**
**Cabinet Regimbeau 26, Avenue Kléber**
**F-75116 Paris (FR)**

**EP 0 012 674 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

# Procédé de synthèse de la vinblastine

La présente invention concerne un procédé de préparation de la vinblastine.
La vinblastine répond à la formule I :

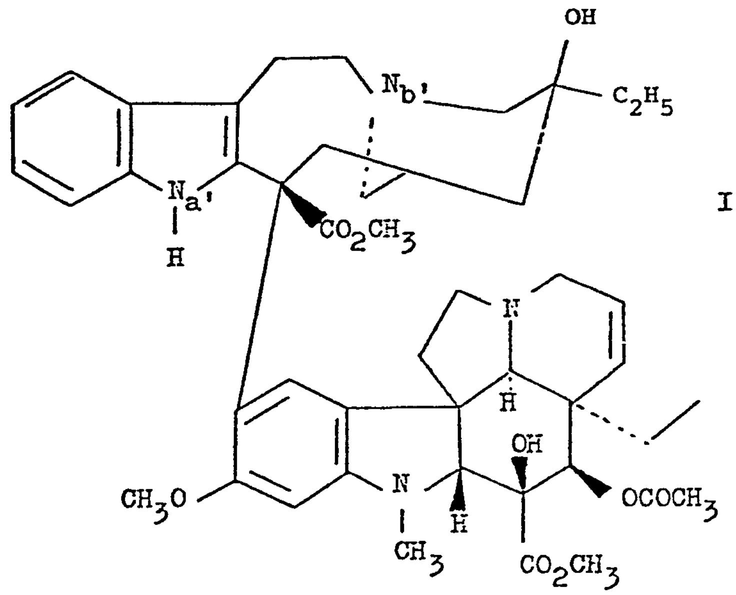

I

Cet alcaloïde peut être isolé de plusieurs espèces de *Catharanthus,* en particulier *C. roseus.* Toutefois, cet alcaloïde qui présente des propriétés antitumorales remarquables n'est présent dans la plante qu'en faible quantité, aussi est-il particulièrement intéressant de pouvoir préparer ce composé par hémisynthèse à partir d'alcaloïdes plus abondants.

L'article de Tetrahedron Letters n° 14, pp. 1099-1102, 1976 décrit la synthèse de la leurosidine, qui est un alcaloïde voisin de la vinblastine, à partir de la $\Delta^{20'}$ déshydroxy-20' vincaleucoblastine par hydroxylation à l'aide de tétraoxyde d'osmium. Toutefois, dans cette synthèse la $\Delta^{20'}$ déshydroxy-20' vincaleucoblastine est préparée par couplage du N-oxyde de la dihydro-15,20S-catharantine avec la vindoline, ce qui conduit à un ion immonium en équilibre avec l'énamine, ce qui conduit dans les étapes subséquentes à des mélanges de produits.

La présente invention se propose de péparer la vinblastine par hydroxylation de la $\Delta^{20'}$ déshydroxy-20' vincaleucoblastine à l'aide d'un agent d'hydroxylation différent du tétroxyde d'osmium et en préparant le produit mentionné précédemment par une réaction différente qui permet de n'obtenir que la forme énamine.

La présente invention concerne donc un procédé de préparation de la vinblastine, caractérisé en ce que l'on traite le $N_b$'-oxyde de déshydroxy-20' leurosidine par un anhydride d'acide carboxylique dans le dichlorométhane à une température comprise entre − 5 et + 5 °C, pour obtenir la $\Delta^{20'}$ déshydroxy-20' vincaleucoblastine qui est hydroxylée dans un solvant chloré en présence d'un sel métallique choisi parmi les sels de $Tl^{3+}$, $Hg^{2+}$ et $Pb^{4+}$, et en ce que l'on réduit le composé obtenu par un borohydrure alcalin dans un solvant hydroxylé.

Les sels de thallium trivalent, les sels de mercure divalent et les sels de plomb tétravalent sont en particulier sous forme de sel d'acide organique, en particulier d'acide carboxylique. Parmi les sels métalliques préférés, il faut mentionner tout particulièrement l'acétate et le trifluoroacétate de thallium trivalent.

L'hydroxylation est conduite dans un solvant chloré tel que le dichlorométhane.

La réaction d'hydroxylation peut être conduite à température ambiante, de préférence sous agitation et sous argon, pendant une durée de quelques heures.

L'étape de réduction du procédé selon la présente invention est conduite en présence d'un borohydrure alcalin, par exemple le borohydrure de sodium, et dans un solvant hydroxylé, par exemple le méthanol ou l'éthanol.

Cette réduction est conduite à une température comprise entre − 5 et + 5 °C, de préférence 0 °C.

Selon la présente invention, la $\Delta^{20'}$ déshydroxy-20' vincaleucoblastine est préparée par action d'un réactif formateur d'ion immonium sur le $N_b$'-oxyde de déshydroxy-20' leurosidine de formule III :

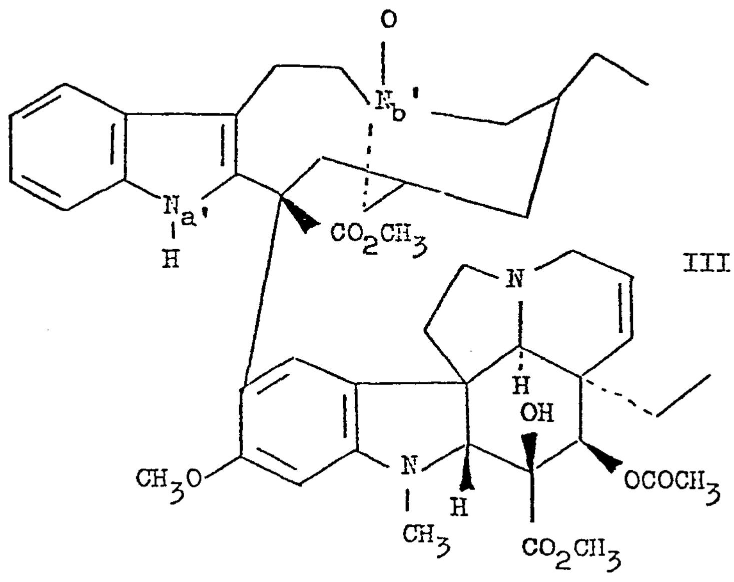

III

Les réactifs formateurs d'ion immonium sont les anhydrides d'acide carboxylique halogéné ou non et parmi ceux-ci il faut citer plus particulièrement l'anhydride acétique.

Dans un mode de mise en œuvre préféré du procédé, le $N_b'$-oxyde en solution dans le dichlorométhane est traité par l'anhydride acétique à une température comprise entre − 5 et + 5 °C afin de fournir la **$\Delta^{20'}$ déshydroxy-20' vincaleucoblastine.**

Le $N_b'$-oxyde peut être préparé par des procédés connus à partir de la déshydroxy-20' leurosidine, en particulier par action d'un peracide organique de formule $R{-}CO_3H$ dans laquelle R est un radical alcoyle ou un radical aryle non substitué ou substitué, le solvant utilisé étant de préférence choisi parmi le dichlorométhane, le chloroforme ou le dichloro-1,2-éthane. Parmi les peracides de formule $R{-}CO_3H$ qui peuvent être utilisés dans la mise en œuvre de cette étape du procédé selon la présente invention on peut citer l'acide p-nitroperbenzoïque, l'acide m-chloroperbenzoïque et l'acide peracétique.

**La déshydroxy-20' leurosidine peut être préparée à partir de la déshydroxy-20' $\Delta^{15'}$ vincaleucoblastine**, encore appelée anhydrovinblastine, par hydrogénation de ce composé. L'hydrogénation de l'anhydrovinblastine peut être effectuée de façon catalytique, par exemple à l'aide d'un métal de transition tel que le palladium sur charbon en suspension dans un solvant organique tel que l'éthanol, sous atmosphère d'hydrogène.

L'anhydrovinblastine est un produit connu dont un procédé d'hémisynthèse a été décrit récemment dans le brevet FR-A-2 296 418.

Ce procédé d'hémisynthèse de l'anhydrovinblastine part de deux alcaloïdes connus, la catharanthine et la vindoline.

La présente invention permet donc la synthèse de la vinblastine à partir de deux alcaloïdes assez abondants dans la nature.

Le schéma ci-après rappelle la synthèse selon la présente invention.

Catharanthine + vindoline

↓

Anhydrovinblastine

↓

Déshydroxy-20' leurosidine

↓

$N_b'$-oxyde déshydroxy-20' leurosidine

↓

**$\Delta^{20'}$ déshydroxy-20' vincaleucoblastine**

↓

Vinblastine

Bien entendu, le procédé selon la présente invention conduit en général à l'obtention d'un mélange de produits dont on isole la vinblastine par des procédés connus, en particulier par chromatographie.

Les exemples suivants sont donnés à titre illustratif et ne sauraient bien entendu limiter aucunement la présente invention.

Exemple 1

Préparation de la déshydroxy-20' leurosidine

A une solution de $\Delta^{15'}$ déshydroxy-20' vincaleucoblastine (100 mg) dans 5 ml d'éthanol, on ajoute du palladium sur charbon à 10 % (10 mg). Le milieu réactionnel est placé sous agitation et sous atmosphère d'hydrogène pendant 12 heures.

Après filtration sur filtre en fibres de verre, le milieu réactionnel est évaporé sous vide et fournit quantitativement la déshydroxy-20' leurosidine.

Exemple 2

Préparation du $N_b'$-oxyde de déshydroxy-20' leurosidine

A une solution de déshydroxy-20' leurosidine (100 mg) dans 5 ml de dichlorométhane sec sous azote et sous agitation, on ajoute l'acide m-chloroperbenzoïque (22 mg). Après 10 mn, la solution est extraite par du chloroforme (50 ml) et lavée 3 fois avec une solution de bicarbonate de sodium dans l'eau (40 g/l) (3 fois 5 ml). Après séchage sur sulfate de sodium, filtration et évaporation, on obtient quantitativement le $N_b'$-oxyde de déshydroxy-20' leurosidine.

Exemple 3

Préparation de la vinblastine

A une solution de $N_b'$-oxyde de déshydroxy-20' leurosidine (50 mg) dans le dichlorométhane (1 ml) on ajoute (150 μl) l'anhydride acétique à 0 °C sous agitation. Après 3 heures, le milieu réactionnel est évaporé à sec sous vide et traité par une solution de triacétate de thallium (50 mg) dans le dichlorométhane (2 ml) à température ambiante sous agitation et sous argon pendant 3 heures.

Le milieu réactionnel est évaporé sous vide, repris par du méthanol (2 ml) et réduit par un excès de borohydrure de sodium. Le milieu réactionnel après réduction est extrait par du chloroforme (50 ml), lavé par de l'eau saturée de chlorure de sodium (2 fois 10 ml), séché sur sulfate de sodium, filtré et évaporé sous vide. La vinblastine ainsi obtenue est séparée des autres produits de la réaction par chromatographie sur couche épaisse de silice (éluant : ACOEt-MeOH 90/10).

**Revendications**

1. Procédé de préparation de la vinblastine, caractérisé en ce que l'on traite le $N_b'$-oxyde de déshydroxy-20' leurosidine par un anhydride d'acide carboxylique dans le dichlorométhane à une température comprise entre − 5 et + 5 °C, pour obtenir la $\Delta^{20'}$ déshydroxy-20' vincaleucoblastine qui est hydroxylée dans un solvant chloré en présence d'un sel métallique choisi parmi les sels de $Tl^{3+}$, $Hg^{2+}$ et $Pb^{4+}$, et en ce que l'on réduit le composé obtenu par un borohydrure alcalin dans un solvant hydroxylé.

2. Procédé selon la revendication 1, caractérisé en ce que le sel est un sel d'acide carboxylique.

3. Procédé selon l'une des revendications 1 et 2, caractérisé en ce que le sel utilisé est l'acétate ou le trifluoroacétate de Tl (III).

4. Procédé selon l'une des revendications 1 à 3, caractérisé en ce que le solvant chloré est le dichlorométhane.

5. Procédé selon l'une des revendications 1 à 4, caractérisé en ce que le $N_b'$-oxyde de déshydroxy-20' leurosidine est traité par l'anhydride d'acide acétique.

6. Procédé selon l'une des revendications 1 à 5, caractérisé en ce que le $N_b'$-oxyde de déshydroxy-20' leurosidine est préparé par action d'un peracide organique sur la déshydroxy-20' leurosidine dans un solvant.

7. Procédé selon la revendication 6, caractérisé en ce que la déshydroxy-20' leurosidine est traitée par l'acide m-chloroperbenzoïque, l'acide p-nitroperbenzoïque ou peracétique dans le dichlorométhane, le chloroforme ou le dichloro-1,2-éthane.

8. Procédé selon l'une des revendications 1 à 7, caractérisé en ce que le solvant hydroxylé est choisi parmi le méthanol et l'éthanol.

9. Procédé selon l'une des revendications 1 à 8, caractérisé en ce que la réduction est conduite à 0 °C.

**Claims**

1. A process for the preparation of vinblastine, characterised in that 20'-dehydroxy leurosidine $N_b'$ oxide is treated with a carboxylic acid anhydride in dichloromethane at a temperature of from − 5 to + 5 °C to obtain $\Delta^{20'}$ 20'-dehydroxy vincaleucoblastine which is hydroxylated in a chlorinated solvent in the presence of a metallic salt selected from among the salts of $Tl^{3+}$, $Hg^{2+}$ and $Pb^{4+}$, and the resulting compound is reduced using an alkaline borohydride in a hydroxyl solvent.

2. A process according to claim 1, characterised in that the salt is a carboxylic acid salt.

3. A process according to one of claims 1 and 2, characterised in that the salt which is used is Tl (III) acetate or trifluoroacetate.

4. A process according to one of claims 1 to 3, characterised in that the chlorinated solvent is dichloromethane.

5. A process according to one of claims 1 to 4, characterised in that 20'-dehydroxy leurosidine $N_b'$ oxide is treated with acetic acid anhydride.

6. A process according to one of claims 1 to 5, characterised in that 20'-dehydroxy leurosidine $N_b'$ oxide is prepared by the action of an organic peracid on 20'-dehydroxy leurosidine in a solvent.

7. A process according to claim 6, characterised in that 20'-dehydroxy leurosidine is treated with m-chloroperbenzoic acid, p-nitroperbenzoic acid or peracetic acid in dichloromethane, chloroform or 1,2-dichloroethane.

8. A process according to one of claims 1 to 7, characterised in that the hydroxyl solvent is selected from among methanol and ethanol.

9. A process according to one of claims 1 to 8, characterised in that reduction is carried out at 0 °C.

**Ansprüche**

1. Verfahren zur Herstellung von Vinblastin, dadurch gekennzeichnet, daß man das $N_b'$-Oxid von 20'-Déhydroxyleurosidin mit einem Anhydrid einer Carbonsäure in Dichlormethan bei einer Temperatur von − 5 bis + 5 °C zum Erhalt des $\Delta^{20'}$-20'-Dehydroxyvincaleucoblastin behandelt, das in einem chlorierten Lösungsmittel in Gegenwart eines Metallsalzes der Metalle $Tl^{3+}$, $Hg^{2+}$ und $Pb^{4+}$ hydroxyliert wird, und daß man das erhaltene Produkt in einem Alkaliborhydrid in einem hydroxylierten Lösungsmittel reduziert.

2. Verfahren nach Anspruch 1, dadurch gekennzeichnet, daß man als Salz ein Carbonsäuresalzverwendet.

3. Verfahren nach einem der Ansprüche 1 und 2, dadurch gekennzeichnet, daß man als Salz das Acetat oder das Trifluoracetat von Tl (III) verwendet.

4. Verfahren nach einem der Ansprüche 1 bis 3, dadurch gekennzeichnet, daß das chlorierte Lösungsmittel Dichlormethan ist.

5. Verfahren nach einem der Ansprüche 1 bis 4, dadurch gekennzeichnet, daß man das $N_b'$-Oxid von 20'-Dehydroxyleurosidin mit Essigsäureanhydrid behandelt.

6. Verfahren nach einem der Ansprüche 1 bis 5, dadurch gekennzeichnet, daß man das $N_b'$-Oxid von 20'-Dehydroxyleurosidin durch Einwirkung einer organischen Persäure auf das 20'-Dehydroxyleurosidin in einem Lösungsmittel herstellt.

7. Verfahren nach Anspruch 6, dadurch gekennzeichnet, daß man das 20'-Dehydroxyleurosidin mit m-Chlorperbenzoesäure, p-Nitroperbenzoesäure oder Peressigsäure in Dichlormethan, Chloroform oder 1,2-Dichloröthan behandelt.

8. Verfahren nach einem der Ansprüche 1 bis 7, dadurch gekennzeichnet, daß man als hydroxyliertes Lösungsmittel Methanol oder Äthanol wählt.

9. Verfahren nach einem der Ansprüche 1 bis 8, dadurch gekennzeichnet, daß man die Reduktion bei 0 °C durchführt.